# EUROPEAN PATENT APPLICATION

(11) **EP 3 854 401 A1**
(43) Date of publication of application: **28.07.2021**
(21) Application number: 19862056.9
(22) Date of filing: 23.09.2019
(51) Int. Cl.: A61K 31/52, A61K 31/519, C07D 473/38, C07D 487/04, A61P 25/28

(54) **CDC7-INHIBITING PURINE DERIVATIVES AND THEIR USE FOR THE TREATMENT OF NEUROLOGICAL CONDITIONS**

(30) Priority: 21.09.2018 ES 201830914
(71) Applicant: Consejo Superior de Investigaciones Científicas (CSIC), 28006 Madrid (ES)
(72) Inventor: MARTÍNEZ GIL, Ana, 28040 Madrid (ES); GIL AYUSO-GONTAN, Carmen, 28040 Madrid (ES); MARTÍN REQUERO, Angeles, 28040 Madrid (ES); ROJAS PRATS, Elisa, 28040 Madrid (ES); MARTINEZ-GONZALEZ, Loreto, 28040 Madrid (ES)
(74) Representative: Pons
(86) International application number: PCT/ES2019/070632
(87) International publication number: WO 2020/058558

(57) **Abstract**

The present invention relates to the use of a series of purine derivatives having the following general formula: 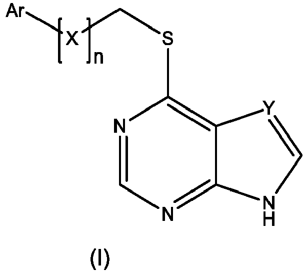 (I) and which are capable of inhibiting CDC7 kinase activity and, therefore, suitable for use in the treatment of neurological diseases such as, inter alia, Alzheimer's disease, amyotrophic lateral sclerosis or frontotemporal dementia, which involve hyperphosphorylation of TDP-43 and the subsequent formation of clusters, induced by CDC7.

## Description

The present invention relates to the use of a series of purine derivatives which are capable of inhibiting CDC7 kinase activity, and, therefore, suitable for use in the treatment and/or prevention of neurological diseases such as amyotrophic lateral sclerosis, Alzheimer's disease or frontotemporal dementia, which involve hyperphosphorylation of TDP-43 and the subsequent formation of clusters, induced by CDC7.

### STATE OF THE ART

Kinase hyperactivity occurs in many types of diseases and particularly in neurodegenerative diseases and cancer. The hyperphosphorylation of the TDP-43 protein induces the formation of clusters which have been detected in patients with amyotrophic lateral sclerosis or frontotemporal lobar degeneration. It has been detected that CDC7 kinase is responsible for the dual hyperphosphorylation of TDP-43 at serines 409/410 in certain models; therefore, the inhibition of this CDC7 would be an interesting strategy for developing drugs for neurodegenerative diseases, such as amyotrophic lateral sclerosis (ALS) or frontotemporal lobar degeneration (Lianchko, N. F. et al., Ann Neurol. 2013 74(1): 39-52). There are other neurological diseases also mediated by TDP-43, such as chronic traumatic encephalopathy and age-related cognitive decline (Iverson GL, et al., Neurosci Biobehav Rev. 2015 Sep; 56: 276-293; Nag S, et al., Neurology. 2017 Feb 14; 88(7): 653-660; Wilson RS, et al., JAMA Neurol. 2013 Nov; 70(11): 1418-1424).

Document US2013/0072506A1 describes 6,8-disubstituted purine derivatives which can be used in a series of therapeutic and cosmetic uses. Among the possible therapeutic uses, the treatment of multiple sclerosis or as antineurodegenerative drugs is mentioned.

Document WO2007/124288A1 describes a series of compounds with an indazole structural core which have the ability to inhibit CDC7 and can be used for the treatment of a disease in which this kinase is involved, such as cancer.

In ACS Med. Chem. Lett. 2013, 4, 211-215, Penning et al. describe a study on the interaction of azaindole-derived compounds with Cdc7 and the possibility of using these compounds in therapy against cancer.

Document US2011/015172A1 describes a family of pyrrolopyrazines which are inhibitors of kinases such as CDC7 and the use thereof for the treatment of diseases associated with this kinase, such as cancer.

### DESCRIPTION OF THE INVENTION

The present invention provides a series of purine-derived compounds which are CDC7 inhibitors and used as potential drugs for diseases mediated by TDP-43 proteinopathies, such as Alzheimer's disease, amyotrophic lateral sclerosis (ALS) and frontotemporal dementia. Particularly, in pathologies with TDP-43 post-translational modifications. These compounds are CDC7 inhibitors in the phosphorylation of TDP-43.

Therefore, in a first aspect, the present invention relates to the use of a compound of formula (I) 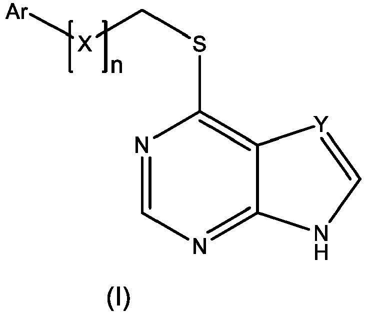 wherein:
X is selected from CH₂, CO;
n is 1;
Y is selected from N, CH;
Ar is selected from the following groups:

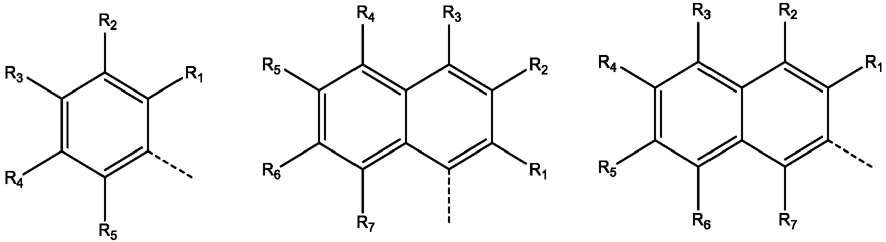

wherein R₁ to R₇ are independently selected from H, NH₂, -O(C₁-C₄ alkyl), NH(C₁-C₄ alkyl) or halogen and ------- represents the binding site to the rest of the molecule;
or wherein n is 0;
Y is CH; and
Ar is the following group:

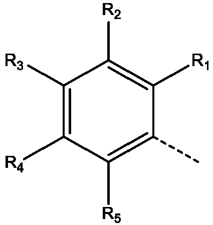

wherein R₁ to R₅ are independently selected from H, NH₂, -O(C₁-C₄ alkyl), NH(C₁-C₄ alkyl) or halogen and ------- represents the binding site to the rest of the molecule; preferably R₁ to R₅ are independently selected from H, NH₂, -O(C₁-C₄ alkyl) or NH(C₁-C₄ alkyl), more preferably R₁ to R₅ are independently selected from H, or -O(C₁-C₄ alkyl);
or any of the pharmaceutically acceptable salts, solvates or isomers thereof for use in the treatment and/or prevention of pathologies related to the TDP-43 protein;
with the condition that the compound of formula (I) is not the following compound:

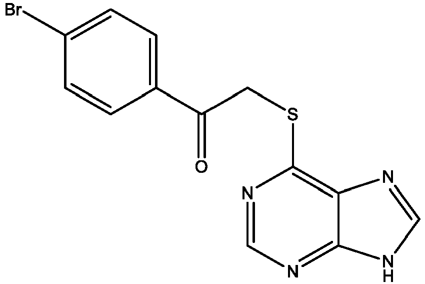

The term "alkyl" refers, in the present invention, to saturated, linear or branched hydrocarbon chains, having 1 to 10 carbon atoms, for example, methyl, ethyl, n-propyl, *i*-propyl, *n*-butyl, *tert*-butyl, sec-butyl, n-pentyl, n-hexyl, etc. Preferably, the alkyl group has between 1 and 4 carbon atoms. The alkyl groups can be optionally substituted by one or more substituents such as CF₃, C₁-C₆ alkyl, C₁-C₆ S-alkyl, halogen, CN, C₁-C₆ O-alkyl, NO₂, C₁-C₆ COO-alkyl, C₁-C₆ NHCO-alkyl, NH₂ and C₁-C₆ NH-alkyl, and more preferably between CF₃, halogen, CN and NO₂.

"Halogen" is understood in the present invention as a bromine, chlorine, iodine or fluorine atom.

In a preferred embodiment of this first aspect, in the compound for use as described, Ar is the following group:

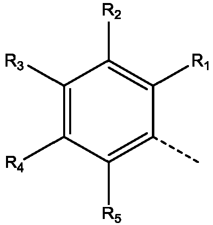

In another more preferred embodiment, in the compound for use as described, at least one of R₁ to R₅ is -O(C₁-C₄ alkyl), preferably O-methyl.

In another more preferred embodiment, in the compound for use as described, at least one of R₁ to R₅ is halogen.

In another more preferred embodiment, in the compound for use as described, R₁ to R₅ is hydrogen.

In a more preferred embodiment, in the compound for use as described, n is 1.

In another more preferred embodiment, in the compound for use as described, X is CO and Y is N.

In another more preferred embodiment, in the compound for use as described, X is CH₂ and Y is N.

In another preferred embodiment, in the compound for use as described, Ar is the following group:

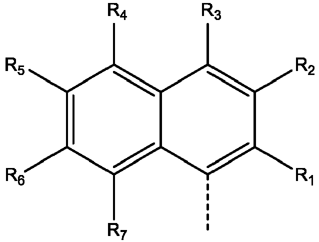

In another more preferred embodiment, in the compound for use as described, X is CO and Y is N.

In another more preferred embodiment, in the compound for use as described above, R₁ to R₇ is hydrogen.

In another more preferred embodiment, in the compound for use as described, at least one of R₁ to R₇ is -O(C₁-C₄ alkyl), preferably it is -O-methyl.

In another preferred embodiment, in the compound for use as described above, at least one of R₁ to R₇ is halogen.

In another preferred embodiment, the compound for use as described is selected from the following list:
- 1-(4-methoxyphenyl)-2-((9*H*-purin-6-yl)thio)-ethan-1-one (2)
- 1-(naphthalen-2-yl)-2-((9*H*-purin-6-yl)thio)-ethan-1-one (3)
- 1-(3-methoxyphenyl)-2-((9*H*-purin-6-yl)thio)-ethan-1-one (4)
- 1-(4-iodophenyl)-2-((9*H*-purin-6-yl)thio)-ethan-1-one (5)
- 6-((4-chlorophenethyl)thio)-9*H*-purine (6)
- 6-((3-chlorophenethyl)thio)-9*H*-purine (7)
- 6-((2-chlorophenethyl)thio)-9*H*-purine (8)
- 6-((4-fluorophenethyl)thio)-9*H*-purine (9)
- 4-(benzylthio)-7*H*-pyrrolo[2,3-*d*]pyrimidine (10)

In another preferred embodiment, the disease related to the TDP-43 protein is a neurological disease. In a more preferred embodiment, the disease related to the TDP-43 protein is a neurological disease selected from amyotrophic lateral sclerosis, frontotemporal dementia, Alzheimer's disease, age-related cognitive decline and chronic traumatic encephalopathy. In an even more preferred embodiment, the disease related to the TDP-43 protein is selected from amyotrophic lateral sclerosis, frontotemporal dementia and Alzheimer's disease.

In a second aspect, the invention relates to a compound which is selected from:
- 6-((4-Chlorophenethyl)thio)-9*H*-purine (6)
- 6-((3-Chlorophenethyl)thio)-9*H*-purine (7)
- 6-((2-Chlorophenethyl)thio)-9*H*-purine (8)
- 6-((4-Fluorophenethyl)thio)-9*H*-purine (9)
- 4-(benzylthio)-7*H*-pyrrolo[2,3-*d*]pyrimidine (10)

In a third aspect, the invention relates to a pharmaceutical composition comprising a compound of the aforementioned second aspect of the invention, together with a pharmaceutically acceptable vehicle. Optionally, this composition may contain another active ingredient.

The compounds of the present invention represented by formula (I) or by the compounds of the second aspect of the invention can include isomers, depending on the presence of multiple bonds (for example, Z, E), including optical isomers or enantiomers, depending on the presence of chiral centres. The individual isomers, enantiomers or diastereoisomers and mixtures thereof fall within the scope of the present invention; in other words, the term isomer also refers to any mixture of isomers, such as diastereomers, racemic isomers, etc., including the optically active isomers thereof or mixtures in different proportions thereof. The individual enantiomers or diastereoisomers, as well as the mixtures thereof, can be separated by conventional techniques.

All the compounds described in the invention can be in crystalline form as free compounds or as solvates. In this sense, the term "solvate", as used here, includes both pharmaceutically acceptable solvates, in other words, solvates of the compound of formula (I) or of the compounds of the second aspect which can be used in the preparation of a medicine, and pharmaceutically unacceptable solvates, which can be used in the preparation of pharmaceutically acceptable salts or solvates. The nature of the pharmaceutically acceptable solvate is not critical as long as it is pharmaceutically acceptable. In a particular embodiment, the solvate is a hydrate. The solvates can be obtained by conventional solvation methods known to those skilled in the art.

For the application thereof in therapy, the compounds of formula (I) or the compounds of the second aspect of the present invention, the salts, solvates or isomers thereof, will be found, preferably, in a pharmaceutically acceptable or substantially pure form, in other words, having a pharmaceutically acceptable purity level excluding normal pharmaceutical additives such as diluents and carriers, and not including material considered toxic at normal dosage levels. The purity levels for the active ingredient are preferably greater than 50%, more preferably greater than 70%, and still more preferably greater than 90%. In a preferred embodiment, they are greater than 95% of the compound of formula (I), (II) or the compounds of the second aspect of the present invention, or the salts, solvates or isomers thereof.

In another aspect, the present invention relates to pharmaceutical compositions comprising at least one compound of the invention, or an isomer, a pharmaceutically acceptable salt or a derivative thereof, together with a pharmaceutically acceptable carrier, an excipient or a vehicle, for the administration to a patient.

The pharmaceutically acceptable adjuvants and vehicles that may be used in said compositions are the adjuvants and vehicles known to those skilled in the art and are usually used to prepare therapeutic compositions.

Another aspect of the invention is a method for treating a neurological or neurodegenerative disease, comprising administering to a patient a therapeutically effective amount of a compound of formula (I), of a compound of the second aspect of the invention or of a pharmaceutical composition comprising them, wherein the neurological or neurodegenerative disease is a disease related to the TDP-43 protein which can be selected mainly from amyotrophic lateral sclerosis, frontotemporal dementia, Alzheimer's disease, age-related cognitive decline and chronic traumatic encephalopathy.

In the sense used in this description, the expression "therapeutically effective amount" refers to the amount of the agent or compound capable of developing the therapeutic action determined by the pharmacological properties thereof, calculated to produce the desired effect and, in general, it will be determined, among other causes, by the very features of the compounds, including the age, state of the patient, the severity of the illness or disorder, and the route and frequency of administration.

The compounds described in the present invention, the salts or solvates thereof, as well as the pharmaceutical compositions containing them can be used together with other additional drugs, or active ingredients, in order to provide a combination therapy. Said additional drugs can make up part of the same pharmaceutical composition or, alternatively, they can be provided in the form of a separate composition for the administration thereof which is simultaneous or not to that of the pharmaceutical composition comprising a compound of formula (I), or a salt or solvate thereof.

In another particular embodiment, said therapeutic composition is prepared in the form of a solid form or aqueous suspension, in a pharmaceutically acceptable diluent. The therapeutic composition provided by this invention can be administered by any suitable route of administration, for which said composition will be formulated in the pharmaceutical form suitable for the chosen route of administration. In a particular embodiment, the administration of the therapeutic composition provided by this invention is performed by the following routes: oral, topical, rectal or parenteral (including subcutaneous, intraperitoneal, intradermal, intramuscular, intravenous, etc.).

In a preferred embodiment of the present invention, the pharmaceutical compositions are suitable for oral administration, in solid or liquid form. The possible forms for oral administration are tablets, capsules, syrups or solutions and may contain conventional excipients known in the pharmaceutical field, such as aggregating agents (e.g., syrup, acacia, gelatin, sorbitol, tragacanth or polyvinylpyrrolidone), fillers (e.g., lactose, sugar, corn starch, calcium phosphate, sorbitol or glycine), disintegrants (e.g., starch, polyvinylpyrrolidone or microcrystalline cellulose) or a pharmaceutically acceptable surfactant such as sodium lauryl sulphate.

The compositions for oral administration can be prepared by conventional galenic pharmacy methods, such as mixing and dispersing. The tablets can be coated following methods known in the pharmaceutical industry.

The pharmaceutical compositions can be adapted for parenteral administration, as sterile solutions, suspensions, or lyophilised solutions of the products of the invention, using the suitable dose. Suitable excipients can be used, such as pH buffering agents or surfactants.

The aforementioned formulations can be prepared using conventional methods, such as those described in the Pharmacopoeias of different countries and in other reference texts.

The administration of the compounds or compositions of the present invention can be performed by means of any suitable method, such as intravenous infusion and the oral, intraperitoneal or intravenous routes. Oral administration is preferred for the convenience of the patients and for the chronic nature of the diseases to be treated.

The administered amount of a compound of the present invention will depend on the relative efficacy of the chosen compound, the severity of the disease to be treated and the weight of the patient. However, the compounds of this invention will be administered one or more times a day, for example 1, 2, 3 or 4 times daily, with a total dose between 0.1 and 1000 mg/Kg/day. It is important to note that it may be necessary to introduce variations in the dose, depending on the age and condition of the patient, as well as modifications in the route of administration.

The compounds and compositions of the present invention can be used together with other drugs in combination therapies. The other drugs can make up part of the same composition or of a different composition, for the administration thereof at the same time or at different times.

The use of the compounds of the invention is compatible with the use thereof in protocols wherein the compounds of formula (I), or the mixtures thereof, are used by themselves or in combinations with other treatments or any medical procedure.

Throughout the description and the claims, the word "comprises" and its variants are not intended to exclude other technical features, additives, components or steps. For those skilled in the art, other objects, advantages and features of the invention may be partially deduced from both the description and the embodiment of the invention. The following examples and figures are provided by way of illustration, and are not intended to limit the present invention.

### BRIEF DESCRIPTION OF THE FIGURES

**FIG. 1** shows the linear correlation between the described permeability and the experimental permeability of 10 commercial compounds using the PAMPA methodology.
**FIG. 2** shows the neuroprotective effect of the CDC7 inhibitor (compound 3) in SH-SY5Y human neuroblastoma cells previously treated with ethacrynic acid (EA, 20 µM) for 12 hours in the presence or absence of the inhibitor at 10 µM and two reference compounds (GSK-3 inhibitors). The data represents the mean of four different experiments ± SEM (**p* <0.05).

### EXAMPLES

Next, the invention will be illustrated by means of assays carried out by the inventors which demonstrate the effectiveness of the product of the invention.

### Example 1: synthesis of the new compounds of the invention.

### 1-(4-Bromophenyl)-2-((9H-purin-6-yl)thio)-ethan-1 -one (1):

**Product 1** has been synthesised according to the procedure described in Chemische Berichte, 1989, 122(5), 919-924.

### 1-(4-Methoxyphenyl)-2-((9H-purin-6-yl)thio)-ethan-1-one (2):

**Product 2** has been synthesised according to the procedure described in Asian Journal of Chemistry, 2010, 22(1), 689-698.

### 1-(Naphthalen-2-yl)-2-((9H-purin-6-yl)thio)-ethan-1-one (3):

### (Commercially available CAS Registry Number 312281-93-9)

6-mercaptopurine monohydrate (300.0 mg, 1.76 mmol) and K₂CO₃ (243.7 mg, 1.76 mmol) are dissolved in DMF (25 ml). The reaction mixture is stirred for 2 h at room temperature. Then 2-bromo-2'-acetonaphthone (439.2 mg, 1.76 mmol) is added and it is stirred for 78 h at room temperature. The solvent is evaporated under reduced pressure and AcOEt (100 ml) is added. The organic phase is washed with distilled water (3 x 100 ml) by adding a little NaCl. It is dried over anhydrous Mg₂SO₄, filtered and concentrated until it is dry. It is not necessary to purify it by means of column chromatography. In this manner, **product 3** is obtained in the form of a yellow solid (158.6 mg, 28%). **¹H-NMR (300 MHz, DMSO-d*₆*):** δ 13.57 (s, 1H), 8.87 (s, 1H), 8.56 (s, 1H), 8.46 (s, 1H), 8.16 (dd, *J* = 8.0, 4.0 Hz, 1H), 8.10 - 7.98 (m, 3H), 7.75 - 7.60 (m, 2H), 5.18 (s, 2H). **¹³C-NMR (75 MHz, DMSO-d*₆*):** δ 193.9 (1C), 158.2 (1C), 152.0 (1C), 149.9 (1C), 143.8 (1C), 135.8 (1C), 133.8 (1C), 132.8 (1C), 131.1 (1C), 130.3 (2C), 129.5 (1C), 129.1 (1C), 128.4 (1C), 127.8 (1C), 124.5 (1C), 37.0 (1C). **HPLC:** Purity = 95%. **MS (ES):** m/z 321 [M+1]. **M.p.** 202 - 204 °C. **Elemental analysis (C₁₇H₁₂N₄OS)** Calculated: C 63.73%, H 3.78%, N 17.49%, S 10.01%. Found: C 63.56%, H 4.02%, N 17.21%, S 9.89%.

### 1-(3-Methoxyphenyl)-2-((9H-purin-6-yl)thio)-ethan-1-one (4):

### (Commercially available CAS Registry Number 1458134-70-7)

6-mercaptopurine monohydrate (300.0 mg, 1.76 mmol) and K₂CO₃ (243.7 mg, 1.76 mmol) are dissolved in DMF (25 ml). The reaction mixture is stirred for 2 h at room temperature. Then 2-bromo-3'-methoxyacetophenone (403.2 mg, 1.76 mmol) is added and it is stirred for 20 h at room temperature. The solvent is evaporated under reduced pressure and AcOEt (100 ml) is added. The organic phase is washed with distilled water (3 x 100 ml) by adding a little NaCl. It is dried over anhydrous Mg₂SO₄, filtered and concentrated until it is dry. It is not necessary to purify it by means of column chromatography. In this manner, **product 4** is obtained in the form of a brown solid (297.0 mg, 55%). **¹H-NMR (300 MHz, DMSO-d*₆*):** δ 13.53 (s, 1H), 8.54 (s, 1H), 8.44 (s, 1H), 7.66 (dd, J = 8.0, 4.0 Hz, 1H), 7.52 (s, 1H), 7.46 (dd, J = 8.0, 4.0 Hz, 1H), 7.23 (dd, *J* = 8.0, 4.0 Hz, 1H), 5.01 (s, 2H), 3.80 (s, 3H). **¹³C-NMR (75 MHz, DMSO-d*₆*):** δ 193.8 (1C), 160.1 (1C), 158.2 (1C), 151.9 (1C), 149.9 (1C), 143.8 (1C), 137.9 (1C), 130.7 (2C), 121.5 (1C), 120.2 (1C), 113.4 (1C), 56.1 (1C), 37.0 (1C). **HPLC:** Purity = 98%. **MS (ES):** m/z 301 [M+1]. **M.p.** 190 - 192 °C. **Elemental analysis (C₁₄H₁₂N4O₂S)** Calculated: C 55.99%, H 4.03%, N 18.65%, S 10.68%. Found: C 55.87%, H 4.00%, N 18.52%, S 10.39%.

### 1-(4-Iodophenyl)-2-((9H-purin-6-yl)thio)-ethan-1-one (5):

### (Commercially available CAS Registry Number 1460254-51-6)

6-mercaptopurine monohydrate (300.0 mg, 1.76 mmol) and K₂CO₃ (243.7 mg, 1.76 mmol) are dissolved in DMF (25 ml). The reaction mixture is stirred for 2 h at room temperature. Then 2-bromo-4'-iodoacetophenone (571.9 mg, 1.76 mmol) is added and it is stirred for 17 h at room temperature. The solvent is evaporated under reduced pressure and AcOEt (100 ml) is added. The organic phase is washed with distilled water (3 x 100 ml) by adding a little NaCl. It is dried over anhydrous Mg₂SO₄, filtered and concentrated until it is dry. The crude product is purified by means of an automatic column using CH₂Cl₂/MeOH (9:1) as eluent. In this manner, **product** 5 is obtained in the form of a yellow solid (61.8 mg, 9%). **¹H-NMR (300 MHz, DMSO-d*₆*):** δ 13.53 (s, 1H), 8.52 (s, 1H), 8.42 (s, 1H), 7.98 (dd, *J* = 8.0, 4.0 Hz, 2H), 7.85 (dd, *J* = 8.0, 4.0 Hz, 2H), 4.96 (s, 2H). **¹³C-NMR (75 MHz, DMSO-d*₆*):** δ 193.7 (1C), 157.2 (1C), 153.7 (1C), 151.7 (1C), 143.9 (1C), 138.3 (2C), 135.6 (1C), 130.5 (3C), 102.5 (1C), 36.5 (1C). **MS (ES):** m/z 396 [M+1]. **M.p.** 192 - 194 °C. **Elemental analysis (C₁₃H₉IN₄OS)** Calculated: C 39.41%, H 2.29%, N 14.14%, S 8.09%. Found: C 39.20%, H 2.19%, N 13.90%, S 7.74%.

### 6-((4-Chlorophenethyl)thio)-9H-purine (6):

6-mercaptopurine monohydrate (300.0 mg, 1.76 mmol) and K₂CO₃ (243.7 mg, 1.76 mmol) are dissolved in DMF (25 ml). The reaction mixture is stirred for 1 h at room temperature. Then 4-chlorophenethyl bromide (387.0 mg, 1.76 mmol) is added and it is stirred for 2 h and 30 min at room temperature. The solvent is evaporated under reduced pressure and AcOEt (100 ml) is added. The organic phase is washed with distilled water (3 x 100 ml) by adding a little NaCl. It is dried over anhydrous Mg₂SO₄, filtered and concentrated until it is dry. The crude product is purified by means of a chromatographic column using CH₂Cl₂/MeOH (10:1) as eluent. In this manner, **product 6** is obtained in the form of a white solid (315.4 mg, 62%). **¹H-NMR (300 MHz, DMSO-d*₆*):** δ 13.51 (s, 1H), 8.71 (s, 1H), 8.44 (s, 1H), 7.40 - 7.29 (m, 4H), 3.59 (t, *J* = 7.5 Hz, 2H), 3.02 (t, *J* = 7.5 Hz, 2H). **¹³C-NMR (75 MHz, DMSO-d*₆*):** δ 158.2 (1C), 151.5 (1C), 149.6 (1C), 143.3 (1C), 139.1 (1C), 131.0 (1C), 130.5 (2C), 130.0 (1C), 128.3 (2C), 34.4 (1C), 29.0 (1C). **M.p.** 190 - 192 °C. **Elemental analysis (C₁₃H₁₁ClN₄S)** Calculated: C 53.70%, H 3.81%, N 19.27%, S 11.03%. Found: C 53.27%, H 3.79%, N 19.03%, S 10.85%.

### 6-((3-Chlorophenethyl)thio)-9H-purine (7):

6-mercaptopurine monohydrate (300.0 mg, 1.76 mmol) and K₂CO₃ (243.7 mg, 1.76 mmol) are dissolved in DMF (25 ml). The reaction mixture is stirred for 1 h at room temperature. Then 3-chlorophenethyl bromide (387.0 mg, 1.76 mmol) is added and it is stirred for 3 h and 30 min at room temperature. The solvent is evaporated under reduced pressure and AcOEt (100 ml) is added. The organic phase is washed with distilled water (3 x 100 ml) by adding a little NaCl. It is dried over anhydrous Mg₂SO₄, filtered and concentrated until it is dry. The crude product is purified by means of a chromatographic column using CH₂Cl₂/MeOH (20:1) as eluent. In this manner, **product 7** is obtained in the form of a white solid (402.2 mg, 78%). **¹H-NMR (300 MHz, DMSO-d*₆*):** δ 13.51 (s, 1H), 8.71 (s, 1H), 8.42 (s, 1H), 7.39 (t, *J* = 1.6 Hz, 1H), 7.37 - 7.24 (m, 3H), 3.62 (t, *J =* 7.5 Hz, 2H), 3.04 (t, *J=* 7.5 Hz, 2H).**¹³C-NMR (75 MHz, DMSO-d*₆*):** δ 158.5 (1C), 151.5 (1C), 149.2 (1C), 142.9 (1C), 142.6 (1C), 133.0 (1C), 130.4 (1C), 130.2 (1C), 128.5 (1C), 127.4 (1C), 126.4 (1C), 34.7 (1C), 28.8 (1C). **M.p.** 143 - 145 °C. **Elemental analysis (C₁₃H₁₁ClN₄S)** Calculated: C 53.70%, H 3.81%, N 19.27%, S 11.03%. Found: C 53.34%, H 3.80%, N 19.09%, S 11.00%.

### 6-((2-Chlorophenethyl)thio)-9H-purine (8):

6-mercaptopurine monohydrate (300.0 mg, 1.76 mmol) and K₂CO₃ (243.7 mg, 1.76 mmol) are dissolved in DMF (25 ml). The reaction mixture is stirred for 1 h at room temperature. Then 2-chlorophenethyl bromide (387.0 mg, 1.76 mmol) is added and it is stirred for 5 h at room temperature. The solvent is evaporated under reduced pressure and AcOEt (100 ml) is added. The organic phase is washed with distilled water (3 x 100 ml) by adding a little NaCl. It is dried over anhydrous Mg₂SO₄, filtered and concentrated until it is dry. The crude product is purified by means of a chromatographic column using CH₂Cl₂/MeOH (10:1) as eluent. In this manner, **product 8** is obtained in the form of a white solid (250.4 mg, 49%). **¹H-NMR (300 MHz, DMSO-d*₆*):** δ 13.49 (s, 1H), 8.70 (s, 1H), 8.43 (s, 1H), 7.44 (dd, *J* = 6.9, 2.5 Hz, 1H), 7.43 (dd, *J* = 6.9, 2.4 Hz, 1H), 7.33 - 7.22 (m, 2H), 3.62 (dd, *J* = 8.3, 6.6 Hz, 2H), 3.17 (dd, *J* = 8.2, 6.7 Hz, 2H). **¹³C-NMR (75 MHz, DMSO-*d₆*):** δ 157.6 (1C), 151.4 (1C), 150.3 (1C), 143.3 (1C), 137.3 (1C), 133.1 (1C), 131.1 (1C), 129.2 (2C), 128.4 (1C), 127.2 (1C), 32.7 (1C), 27.6 (1C). **M.p.** 154 - 156 °C. **Elemental analysis (C₁₃H₁₁ClN₄S)** Calculated: C 53.70%, H 3.81%, N 19.27%, S 11.03%. Found: C 53.60%, H 3.86%, N 19.14%, S 10.85%.

### 6-((4-Fluorophenethyl)thio)-9H-purine (9):

6-mercaptopurine monohydrate (300.0 mg, 1.76 mmol) and K₂CO₃ (243.7 mg, 1.76 mmol) are dissolved in DMF (25 ml). The reaction mixture is stirred for 1 h at room temperature. Then 4-fluorophenethyl bromide (358.0 mg, 1.76 mmol) is added and it is stirred for 5 h at room temperature. The solvent is evaporated under reduced pressure and AcOEt (100 ml) is added. The organic phase is washed with distilled water (3 x 100 ml) by adding a little NaCl. It is dried over anhydrous Mg₂SO₄, filtered and concentrated until it is dry. The crude product is purified by means of a chromatographic column using CH₂Cl₂/MeOH (10:1) as eluent. In this manner, **product 9** is obtained in the form of a white solid (285.0 mg, 59%). **¹H-NMR (300 MHz, DMSO-d*₆*):** δ 13.51 (s, 1H), 8.70 (s, 1H), 8.42 (s, 1H), 7.45 - 7.26 (m, 2H), 7.22 - 6.94 (m, 2H), 3.59 (t, *J=* 7.6 Hz, 2H), 3.02 (t, *J* = 7.5 Hz, 2H). **¹³C-NMR (75 MHz, DMSO-*d₆*):** δ 160.9 (d, *J* = 241.7 Hz, 1C), 158.6 (1C), 151.5 (1C), 149.2 (1C), 142.9 (1C), 136.3 (d, *J* = 2.7 Hz, 1C), 130.4 (d, *J* = 7.9 Hz, 2C), 130.4 (1C), 115.0 (d, *J* = 21.0 Hz, 2C), 34.3 (1C), 29.2 (1C). **HPLC:** Purity > 99%, t.r. = 6.99 min. **MS (ES):** m/z 275 [M+1]. **M.p.** 184 - 186 °C. **Elemental analysis (C₁₃H₁₁FN₄S)** Calculated: C 56.82%, H 4.04%, N 20.42%, S 11.69%. Found: C 56.38%, H 4.01%, N 20.04%, S 11.43%.

### 4-(benzylthio)-7H-pyrrolo [2,3-d]pyrimidine (10):

Under an argon atmosphere and at 0 °C, a mixture of 6-mercaptopurine (0.49 g, 3.9 mmol) and NaH (47 mg, 1.96 mmol) is added in DMF on a solution of 4-chloro-7H-pyrrolo[2,3-*d*]pyrimidine (0.15 g, 1.0 mmol) in DMF. The reaction mixture is stirred at room temperature under an argon atmosphere overnight. After the reaction is complete, the mixture is neutralised with methanol. The solvent is evaporated under reduced pressure and AcOEt (100 ml) is added. The organic phase is washed with distilled water (3 x 100 ml). It is dried over anhydrous Mg₂SO₄, filtered and concentrated until it is dry. The crude product is purified by means of a chromatographic column using Hexane/AcOEt (10:1) as eluent. In this manner, **product 10** is obtained in the form of a white solid (164 mg; 70%). **¹H-NMR (300 MHz, DMSO-d*₆*):** δ 4.68 (s, 2H, CH2), 6.52 (dd, *J=* 4.0, 2.0 Hz, 1H), 7.26-7.50 (m, 6H), 8.69 (s, 1H), 12.25 (s, 1H, NH). **¹³C-NMR (DMSO-*d₆*):** δ 31.6, 98.2, 114.7, 125.8, 127.0, 128.4, 128.9, 138.0, 149.0, 150.2, 158.7. **M.p.** 167-168 °C **Elemental analysis (C₁₃H₁₁N₃S)** Calculated: C, 64.71; H, 4.59; N, 17.41; S, 13.29. Found: C, 64.59; H, 4.64; N, 17.28; S, 13.22.

### Example 2: Measurement of CDC7 inhibition

The LanthaScreen Eu Kinase Inhibition Assay uses an Alexa Fluor™ marker which binds to a kinase and is detected by the addition of an Eu-labelled antibody. The binding of the marker and the antibody to the kinase results in a high degree of FRET, whereas the displacement of the marker by an inhibitor results in a loss of FRET. Unlike many other kinase activity assays, this is a simple mix and read assay, without stages of development. This assay method has been developed by "Life Technologies" and they identify competitive ATP kinase inhibitors, making them suitable for the detection of any compound which binds to the ATP site.

The compounds are evaluated in 1% DMSO (final) in the well. A mixture of recombinant human Cdc7/DBF4 (0.5 nM), Eu-anti-GST antibody (2 nM) and Alexa Fluor marker (1 nM) in a buffer of 50 mM HEPES pH 7.5, 0.01% BRIJ-35, 10 mM MgCI2, 1 mM EGTA was used. In white plates with 348 wells, with a low volume, and coded (Greiner cat. #784207), 160 nL (100 x compound in 100% DMSO), 3.84 µl (buffer with Cdc7/DBF4), 8.0 µl (antibody), 4.0 µl (marker) are added. It is stirred for 30 s and incubated at room temperature for 60 min. Next, the fluorescence is measured in the plate reader and the data is analysed (Table 1).

**Table 1. Inhibition values in Cdc7 of the compounds of formula (I):**

| **Comp.** | 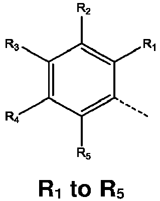 | 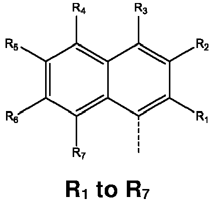 | **X** | **Z** | **Y** | **Cdc7 Cl₅₀ (µM)** |
|---|---|---|---|---|---|---|
| 1* | 4-Br | - | CO | S | N | 2.120 |
| 2 | 4-OMe | - | CO | S | N | 1.080 |
| 3 | - | H | CO | S | N | 0.698 |
| 4 | 3-OMe | - | CO | S | N | 1.710 |
| 5 | 4-I | - | CO | S | N | 0.380 |
| 6 | 4-Cl | - | CH₂ | S | N | 0.142 |
| 7 | 3-Cl | - | CH₂ | S | N | 0.305 |
| 8 | 2-Cl | - | CH₂ | S | N | 0.191 |
| 9 | 4-F | - | CH₂ | S | N | 0.397 |
| 10 | H | - | - | S | CH | 0.234 |

| | | | | | | |
|---|---|---|---|---|---|---|
| *Comp. (1): 1-(4-Bromophenyl)-2-((9*H*-purin-6-yl)thio)-ethan-1-one, included for comparison. | | | | | | |

### Example 3: Prediction of the passage across the blood-brain barrier

An essential requirement that must be met by drugs for the treatment of neurodegenerative diseases is the ability to cross the blood-brain barrier (BBB) since, otherwise, they could not act on the target of interest. Therefore, for the compounds that are non-permeable or located in the zone of uncertainty, it could be necessary for them to be suitably conveyed in a pharmaceutical formulation by means of methods known by those skilled in the art, such as by means of encapsulation. This ability can be predicted *in vitro* by using a method known by the acronym PAMPA (*Parallel Artificial Membrane Permeability Assay*) described by Di et al. (Di, L.; Kerns, E. H.; Fan, K.; McConnell, O. J.; Carter, G. T. Eur. J. Med. Chem., 2003, 38 (3), 223-232) and that has subsequently been fine-tuned in our research group. Said method enables the effective permeability through artificial membranes to be predicted by means of a passive diffusion process.

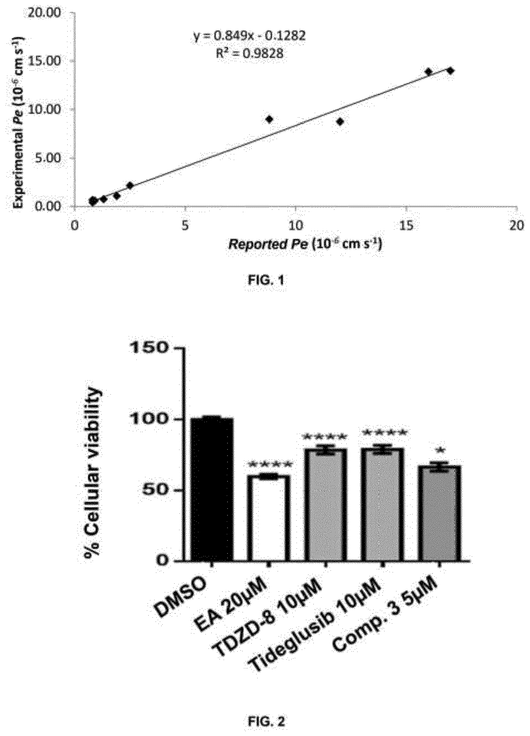

First, it is necessary to validate the method, for which reason 10 commercial compounds, whose ability to penetrate the central nervous system (CNS) is known, are used and will be specified below, obtaining in this case good linear correlation between the experimental permeability values (*Pe*) and those described (FIG. 1). This correlation line obtained following the pattern described in the literature makes it possible to establish the limits for predicting whether a compound can cross the blood-brain barrier or not.

Thus, a compound is considered to be permeable to the BBB (CNS+) if it has a permeability > 4.48 x 10⁻⁶cm·s⁻¹.

For the procedure, between 3-5 mg of caffeine, desipramine, enoxacin, hydrocortisone, ofloxacin, piroxicam and testosterone, 12 mg of promazine and 25 mg of atenolol and verapamil were taken and dissolved in EtOH (1000 µl). 100 µl of these solutions were taken and EtOH (1400 µl) and phosphate buffer (PBS) pH = 7.4 (3500 µl) were added in order to reach a final EtOH concentration of 30% v/v in solution. Lastly, the solutions were filtered.

Moreover, a solution of PBS/EtOH (70:30) was added to each well of the acceptor plate (180 µl). The donor plate was impregnated with a solution of porcine brain lipid (4 µl) dissolved in dodecane (20 mg·ml⁻¹). After 5 min, a solution of each compound was added on this plate (180 µl).

Of the compounds evaluated, between 1-2 mg were taken and dissolved in EtOH (1500 µl) and phosphate buffer (PBS) pH = 7.4 (3500 µl), filtered and added to the donor plate. With these solutions, the wavelengths at which they absorb the compounds are determined and the initial absorbance levels at these wavelengths are measured by using a UV absorbance reader. Each sample was analysed at two to five wavelengths, in three wells and in two independent experiments.

Next, the donor plate was deposited on the acceptor plate forming a sort of "sandwich" and they were left to incubate for 2 h and 30 min at 25 °C. Thus, the compounds will pass from the donor plate to the acceptor plate through the porcine brain lipid by means of passive diffusion. After that time, the donor plate is carefully removed and the final concentration and absorbance of both the commercial and the synthesised compounds are determined. The results obtained are expressed as the mean of the measurements [standard deviation (SD)] of the different experiments performed and are recorded in Table 2.

**Table 2. Permeability values (Pe 10⁻⁶cm s⁻¹) in the PAMPA-BBB experiment and prediction of the penetration into the central nervous system (CNS) of the compounds of formula (II) as they are also described in Table 1:**

| **Comp.** | 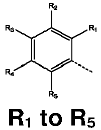 | 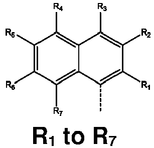 | **X** | **Z** | **Y** | ***Pe* (10⁻⁶ cm s⁻¹)** | **PAMPA prediction** |
|---|---|---|---|---|---|---|---|
| 2 | 4-OMe | - | CO | S | N | 2.5 ± 0.1 | CNS+/- |
| 3 | - | H | CO | S | N | 9.6 ± 0.8 | CNS+ |
| 4 | 3-OMe | - | CO | S | N | 2.4 ± 1 | CNS+/- |
| 5 | 4-I | - | CO | S | N | 10.5 ±1.2 | CNS+ |
| 6 | 4-Cl | - | CH₂ | S | N | 25.1 ± 1.3 | CNS+ |
| 7 | 3-Cl | - | CH₂ | S | N | 10.6 ± 0.5 | CNS+ |
| 8 | 2-Cl | - | CH₂ | S | N | 11.6 ± 0.5 | CNS+ |
| 9 | 4-F | - | CH₂ | S | N | 10.2 ± 0.5 | CNS+ |
| 10 | H | - | - | S | CH | 4.5 ± 1.2 | CNS+ |

### Example 4: Neuroprotective effect of the Cdc7 inhibitors against ethacrynic acid

The SH-SY5Y human neuroblastoma cell line was grown at 37 °C with 5% CO₂ in DMEN (Dulbecco's Modified Eagle Medium) enriched with L-glutamine (2mM), 1% non-essential amino acids, 1% Penicillin/Streptomycin and 10% foetal bovine serum. In the state of semi-confluence, the cells were treated with the Cdc7 inhibitor (compound 3) at different concentrations for 1.30 hours post-addition of the causative agent of the TDP-43 phosphorylation; ethacrynic acid (20 µM) (Sigma). At 24 hours, the cellular viability was evaluated with MTT ([3- (4,5-dimethylthiazol-2-yl) -2,5-diphenyltetrazolium bromide) following a described procedure (Denizot F, Lang R. J Immunol Methods. 1987; 89:271-7). Figure 2 shows how the cells have a 40% mortality due to the excess TDP-43 phosphorylation (ethacrynic acid) and how this death is reversed with the addition of the reference standards, as well as with the CDC7 inhibitor.

## Claims

1. A compound of formula (I) 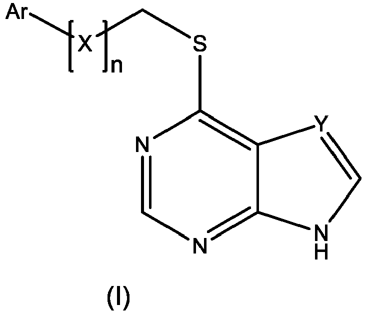 wherein:
X is selected from CH₂, CO;
n is 1;
Y is selected from N, CH;
Ar is selected from the following groups: 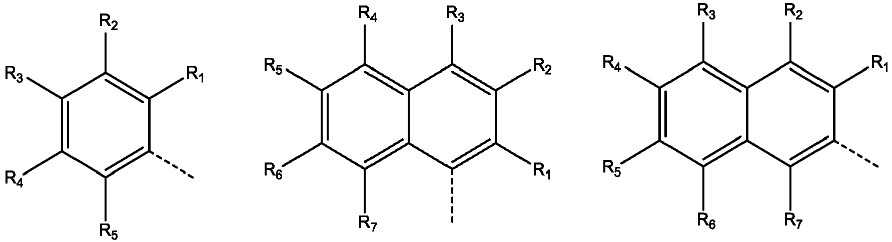
wherein R₁ to R₇ are independently selected from H, NH₂, -O(C₁-C₄ alkyl), NH(C₁-C₄ alkyl) or halogen and ------- represents the binding site to the rest of the molecule;
or wherein n is 0;
Y is CH; and
Ar is the following group: 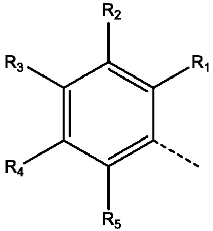
wherein R₁ to R₅ are independently selected from H, NH₂, -O(C₁-C₄ alkyl), NH(C₁-C₄ alkyl) or halogen and ------- represents the binding site to the rest of the molecule;
or any of the pharmaceutically acceptable salts, solvates or isomers thereof for use in the treatment and/or prevention of pathologies related to the TDP-43 protein;
with the condition that the compound of formula (I) is not the following compound:

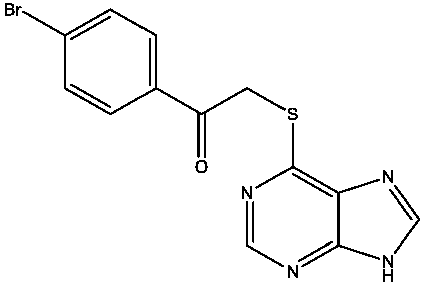

2. The compound for use according to claim 1, wherein Ar is the following group:

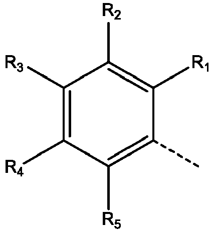

3. The compound for use according to claim 2, wherein n is 1.

4. The compound for use according to claim 3, wherein X is CO and Y is N.

5. The compound for use according to claim 3, wherein X is CH₂ and Y is N.

6. The compound for use according to any of claims 2 to 5, wherein at least one of R₁ to R₅ is -O(C₁-C₄ alkyl).

7. The compound for use according to claim 6, wherein at least one of R₁ to R₅ is -O-methyl.

8. The compound for use according to any of claims 2 to 5, wherein at least one of R₁ to R₅ is halogen.

9. The compound for use according to claim 1, wherein Ar is the following group:

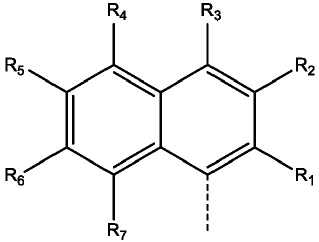

10. The compound for use according to claim 9, wherein X is CO and Y is N.

11. The compound for use according to any of claims 9 to 10, wherein R₁ to R₇ is H.

12. The compound for use according to claim 1, which is selected from the following list:
• 1-(4-methoxyphenyl)-2-((9*H*-purin-6-yl) thio)-ethan-1-one
• 1-(naphthalen-2-yl)-2-((9*H*-purin-6-yl)thio)-ethan-1-one
• 1-(3-methoxyphenyl)-2-((9*H*-purin-6-yl)thio)-ethan-1-one
• 1-(4-iodophenyl)-2-((9*H*-purin-6-yl)thio)-ethan-1-one
• 6-((4-chlorophenethyl)thio)-9*H*-purine
• 6-((3-chlorophenethyl)thio)-9*H*-purine
• 6-((2-chlorophenethyl)thio)-9*H*-purine
• 6-((4-fluorophenethyl)thio)-9*H*-purine
• 4-(benzylthio)-7*H*-pyrrolo[2,3-*d*]pyrimidine.

13. The compound for use according to any of claims 1 to 12, wherein the disease related to the TDP-43 protein is a neurological disease.

14. The compound for use according to claim 13, wherein the disease related to the TDP-43 protein is a neurological disease selected from amyotrophic lateral sclerosis, frontotemporal dementia, Alzheimer's disease, age-related cognitive decline and chronic traumatic encephalopathy.

15. The compound for use according to claim 14, wherein the disease related to the TDP-43 protein is selected from amyotrophic lateral sclerosis, frontotemporal dementia and Alzheimer's disease.

16. A compound selected from:
• 6-((4-Chlorophenethyl)thio)-9*H*-purine
• 6-((3-Chlorophenethyl)thio)-9*H*-purine
• 6-((2-Chlorophenethyl)thio)-9*H*-purine
• 6-((4-Fluorophenethyl)thio)-9*H*-purine
• 4-(benzylthio)-7*H*-pyrrolo[2,3-*d*]pyrimidine.

17. A pharmaceutical composition comprising a compound according to claim 16 together with a pharmaceutically acceptable vehicle.

18. The compound according to claim 16 for use as a medicine.
